# EUROPÄISCHE PATENTSCHRIFT

(11) **EP 3 727 209 B1**
(45) Veröffentlichungstag und Bekanntmachung des Hinweises auf die Patenterteilung: **26.01.2022**
(21) Anmeldenummer: 18826308.1
(22) Anmeldetag: 18.12.2018
(51) Int. Cl.: A61F 2/60, A61F 2/68, A61F 2/70, H01R 25/00

(54) **SYSTEM AUS MEHREREN ORTHOPÄDIETECHNISCHEN KOMPONENTEN SOWIE VERFAHREN ZUR STEUERUNG EINES SOLCHEN SYSTEMS**
SYSTEM MADE UP OF MULTIPLE ORTHOPEDIC COMPONENTS AND METHOD FOR CONTROLLING SUCH A SYSTEM
SYSTÈME CONSTITUÉ DE PLUSIEURS COMPOSANTS ORTHOPÉDIQUES ET PROCÉDÉ DE COMMANDE D'UN TEL SYSTÈME

(30) Priorität: 22.12.2017 DE 102017131190
(43) Veröffentlichungstag der Anmeldung: 28.10.2020
(73) Patentinhaber: Otto Bock Healthcare Products GmbH, 1110 Wien (AT)
(72) Erfinder: PAPPE, Alexander, 1180 Wien (AT); WEIGL-POLLACK, Andreas, 3464 Goldgeben (AT); NOLTE, Michael, 37136 Seeburg (DE); ALBRECHT-LAATSCH, Erik, 37124 Rosdorf (DE); KAITAN, Robert, 1220 Wien (AT); HOFFMANN, Robert, 1160 Wien (AT); PAUSER, Thomas, 7035 Steinbrunn (AT); SCHRAMEL, Andreas, 1160 Wien (AT); SAGMEISTER, Luis, 2823 Pitten (AT); MOSLER, Lüder, 37115 Duderstadt (DE); WÜSTEFELD, Andreas, 37434 Obernfeld (DE); KLINGEBIEL, Karl-Heinz, 37115 Duderstadt (DE)
(74) Vertreter: Gramm, Lins & Partner Patent- und Rechtsanwälte PartGmbB
(86) Internationale Anmeldenummer: PCT/EP2018/085665
(87) Internationale Veröffentlichungsnummer: WO 2019/121794

(56) Entgegenhaltungen:
- WO-A1-2010/064063
- US-A1- 2007 156 252

## Beschreibung

Die Erfindung betrifft ein System aus mehreren orthopädietechnischen Komponenten, die miteinander gekoppelt sind, mit einer ersten elektronischen und/oder elektrischen Einrichtung, die einen ersten Versorgungsanschluss aufweist, über den die erste elektrische und/oder elektronische Einrichtung mit Energie und/oder Daten von einer Ladestation über einen Stecker versorgbar ist. Die Erfindung betrifft ebenfalls ein Verfahren zur Steuerung eines solchen Systems.

Als orthopädietechnische Komponenten werden Orthesen, Prothesen, Rollstühle, Datenlogger, Funkmodule, Feedbackelemente, elektrische Speichereinrichtungen oder Teile von Orthesen oder Prothesen oder Rollstühlen angesehen, beispielsweise Prothesengelenke, Prothesenfüße, Rohradapter, Prothesenhände, Prothesenellenbogen, Drehadapter, Prothesenschäfte, Orthesenschienen, Orthesengelenke, Fußschalen sowie daran angeordnete Sensoren, Speichereinrichtungen, Prozessoren oder andere Datenverarbeitungseinrichtungen. Ein System aus mehreren orthopädietechnischen Komponenten ist eine Kombination solcher Komponenten zu einer orthopädietechnischen Einheit, die eine über die Funktionalität der einzelnen orthopädietechnischen Komponente hinausgehende Funktionalität aufweist. Ein solches System kann beispielsweise eine Oberschenkelprothese aus einem Oberschenkelschaft, ein Prothesenkniegelenk, ein Unterschenkelrohr sowie ein Prothesenfuß sein. Das System einer Unterschenkelprothese besteht aus einem Unterschenkelschaft und einem daran angeordneten Prothesenfuß. Prothesen einer oberen Extremität als Oberarmprothese können einen Schaft, Eingabegeräte, Steuergeräte, Energieversorgungen wie Batterien, Akkumulatoren, Kondensatoren, Transformatoren oder Netzteile, eine Schulter, einen aktiven Ellbogen, ein Handgelenk , Greifgeräte, z.B. einen Greifer und/oder einzelne Finger aufweisen. Ein Orthesensystem kann beispielsweise als eine Knie-Knöchel-Fuß-Orthese (KAFO) ausgebildet sein.

Orthopädietechnische Komponenten sind häufig mit elektrischen und/oder elektronischen Einrichtungen versehen, um beispielsweise wirksame Kräfte, Raumlagen, Momente oder Zuordnungen von Bauteilen zueinander zu detektieren. Darüber hinaus kann auf der Grundlage von ermittelten Sensorwerten, die in einem Prozessor ausgewertet werden, eine Veränderung von Widerständen einer Dämpfereinrichtung oder eine Aktivierung oder Deaktivierung eines motorischen Antriebes erfolgen. Die Nutzungsdaten der orthopädietechnischen Komponente können ausgewertet werden, beispielsweise um für zukünftige Einstellungen eine Datengrundlage zu haben. Darüber hinaus können Funktionsprüfungen durch angeschlossene Prüfeinrichtungen durchgeführt werden. Komplette prothetische oder orthetische Einrichtungen, wie computergesteuerte Armprothesen oder Exoskelette werden für aufwendige Versorgungen benötigt.

Aus der WO 2010/064063 A1 ist eine Prothese mit einem Oberschenkel- und einem Unterschenkelteil sowie einem Fußteil bekannt, die gelenkig miteinander verbunden sind und hydraulische Dämpfer mit zugeordneten Elektromotoren aufweisen. Über einen USB-Steckeranschluss werden Daten elektronischen Komponenten übermittelt und eine Batterie geladen.

Insbesondere bei komplexeren orthopädietechnischen Systemen weist jeder Aktuator oder elektrische Verbraucher einen ihm zugeordneten Energiespeicher auf. Darüber hinaus können die Abstimmungen beispielsweise eines prothetischen Kniegelenkes mit einem aktuierbaren prothetischen Knöchelgelenk aufwendig sein und gegebenenfalls unabhängig voneinander durchgeführt werden. Auch bei nicht angetriebenen Komponenten können elektronische Einrichtungen vorgesehen sein, beispielsweise um Belastungen oder Winkellagen über integrierte Sensoren zu erfassen. Durch den modularen Aufbau und Prothesen oder Orthesen existiert eine Vielzahl unterschiedlicher Kombinationsmöglichkeiten, was die Komplexität bei einem Softwareabgleich oder einer Energieversorgung erhöhen kann.

Aufgabe der vorliegenden Erfindung ist es daher, ein System aus mehreren orthopädietechnischen Komponenten sowie ein Verfahren zur Steuerung eines solchen Systems bereitzustellen, das leichter zu handhaben ist und eine einheitliche Versorgung mit Daten und/oder elektrischer Energie ermöglicht.

Erfindungsgemäß wird diese Aufgabe durch ein System mit den Merkmalen des Hauptanspruches und ein Verfahren mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das erfindungsgemäße System aus mehreren orthopädietechnischen Komponenten, die miteinander gekoppelt sind, mit einer ersten elektronischen und/oder elektrischen Einrichtung, die einen ersten Versorgungsanschluss aufweist, über den die erste elektrische und/oder elektronische Einrichtung mit Energie und/oder Daten von einer Ladestation über einen Stecker versorgbar ist, sieht vor, dass zumindest eine zweite elektrische und/oder elektronische Einrichtung an einer der Komponenten angeordnet ist, die einen separaten zweiten Versorgungsanschluss und/oder einen Steckeranschluss aufweist, der oder die mit der ersten elektronischen und/oder elektrischen Einrichtung zur Übertragung von Energie und/oder Daten über den ersten Versorgungsanschluss oder einen separaten Steckeranschluss koppelbar ist oder sind. Die beiden Komponenten können unterschiedliche Anschlüsse zum Verbinden mit einer Ladestation und zum Verbinden untereinander aufweisen. Die Anforderungen an eine Verbindung eines Versorgungsanschlusses mit einer Ladestation können sich von den Anforderungen an eine Verbindung zwischen zwei häufig langfristig miteinander verbundenen Komponenten unterscheiden. Die Häufigkeit einer Kontaktierung und Dekontaktierung unterscheidet sich ebenso wie die Anforderungen an einen Berührschutz oder eine Wasserdichtigkeit, so dass die Stecker für Versorgungsanschlüsse anders als Stecker für Steckeranschlüsse oder Steckerbuchsen ausgestaltet sein können. Grundsätzlich können Steckeranschlüsse und Versorgungsanschlüsse kompatibel zueinander sein, so dass mit einem Stecker oder einem Steckertyp sowohl eine Verbindung zu einer Ladestation als auch zwischen zwei Komponenten hergestellt werden kann, wobei für unterschiedliche Verbindungen nicht alle Funktionen oder Belegungen aktiviert sein müssen. Der Steckeranschluss kann eine höhere IP-Schutzklasse als der Versorgungsanschluss aufweisen, da der Versorgungsanschluss bei einem nicht gesteckten Kontakt in der Regel potentialfrei ist. Über eine Trennung von Versorgungsanschluss und Steckeranschluss ist es möglich, den unterschiedlichen Anforderungen an einen reinen, in der Regel kurzen Energietransfer mit häufiger Kontaktierung und einer dauerhaften Steckerverbindung mit Energie- und Datentransport Rechnung zu tragen.

Eine Weiterbildung der Erfindung sieht vor, dass die erste und zweite elektronische und/oder elektrische Einrichtung über eine Steckerverbindung zwischen den Steckeranschlüssen, den Versorgungsanschlüssen oder einem Steckeranschluss und einem Versorgungsanschluss miteinander koppelbar sind, wodurch die Möglichkeit besteht, alle Stecker einheitlich auszugestalten. Alternativ sind bei unterschiedlichen Funktionen, insbesondere wenn diese einander ausschließen, unterschiedliche Steckerformen ausgebildet, um eine versehentliche Falschkontaktierung zu vermeiden.

Die Versorgungsanschlüsse der ersten und zweiten elektronischen und/oder elektrischen Einrichtungen können mit dem Steckeranschluss kompatibel sein. Über den zweiten Versorgungsanschluss oder einen Steckeranschluss, der mit dem Stecker zur Versorgung der ersten elektrischen und/oder elektronischen Einrichtung kompatibel ist, ist es möglich, eine einfache Kommunikation zwischen den zumindest zwei orthopädietechnischen Komponenten innerhalb des Systems bereitzustellen. Darüber hinaus ist es möglich, die jeweiligen orthopädietechnischen Komponenten und die darin oder daran angeordneten elektrischen und/oder elektronischen Einrichtungen mit Energie zu versorgen, insbesondere die elektrischen Energiespeicher oder Akkus aufzuladen und darüber hinaus die während des Betriebes erfassten Daten auszulesen und auszuwerten. Über die kompatible Ausgestaltung der Versorgungsanschlüsse und ggf. Steckeranschlüsse ist es zudem möglich, mit nur einer Ladestation alle elektrischen und/oder elektronischen Einrichtungen mit einer Ladestation, über die Energie und/oder Daten zugeleitet werden, zu versorgen. Dadurch werden die zwei oder mehr orthopädietechnischen Komponenten als eine Einheit angesehen, die zentral versorgt und hinsichtlich ihrer speziellen Funktionalität, die auf den jeweiligen Patienten abgestimmt ist, angepasst behandelt werden kann.

Die elektrische und/oder elektronische Einrichtung kann einen Aktuator aufweisen, z.B. einen motorischen Antrieb, einen hydraulischen Antrieb oder Dämpfer, eine Sperre oder eine pneumatische Einrichtung, und/oder eine Steuerungseinrichtung, eine Verarbeitungsschaltung oder Prozessor, einen Sensor, einen Datenspeicher, eine Ladeeinheit und/oder einen Energiespeicher aufweisen. Das Ladegerät oder die Ladeeinheit kann als eines der kommunizierenden Geräte ausgebildet sein und an dem Bussystem teilnehmen, über das alle Komponenten des Systems miteinander kommunizieren.

Bevorzugt sind die orthopädietechnischen Komponenten mechanisch und/oder elektrisch miteinander gekoppelt. Über die mechanische Kopplung der Komponenten miteinander ist es möglich, bei einer gelenkigen Verbindung Verschwenkbewegungen und bei anderen Führungseinrichtungen andere Verlagerungen der Komponenten zueinander zu ermöglichen. Darüber hinaus können Kräfte oder Momente übertragen werden, beispielsweise um Bodenreaktionskräfte beim Gehen aufnehmen und auf den Körper durchleiten zu können. Kräfte und Momente können von einer Komponente auf die andere übertragen werden. Über die mechanische Kopplung ist es möglich, bei einem modularen Aufbau von orthopädietechnischen Komponenten, wie Orthesen, Prothesen oder Rollstühlen, aus vielen Einzelteilen eine an den jeweiligen Patienten angepasste Orthese, Prothese oder andere orthopädietechnische Einrichtung zusammenzustellen.

Eine elektrische Kopplung mit der Ladestation oder zwischen zwei Versorgungsanschlüssen kann beispielsweise über eine Steckerverbindung realisiert werden, bei der zumindest eine Steckerseite ein kaskadierender Stecker ist, der dem Versorgungsanschluss zugewandten Seite eine entsprechende Steckerausbildung aufweist und auf der abgewandten Seite eine Aufnahme oder Buchse für den Stecker, wie er von der Ladestation kommt, ausbildet. Die Verkabelung kann innerhalb des orthopädietechnischen Systems, beispielsweise der Prothese oder der Orthese verlaufen, um die Kabel vor äußeren Einflüssen zu schützen. Ebenso können die Stecker innerhalb des orthopädietechnischen Systems angeordnet sein, wobei die Zugänglichkeit von außen, beispielsweise bei angelegter Prothese oder Orthese gegeben sein kann. Von diesem kaskadierend aufgebauten Stecker zweigt zumindest ein Kabel ab zu einem Stecker, der an den zweiten Versorgungsanschluss anschließbar ist. Über einen solchen Aufbau können zwei oder mehrere orthopädietechnische Komponenten mit Versorgungsanschlüssen miteinander elektrisch und elektronisch miteinander gekoppelt werden, um Daten und/oder Energie einzuleiten oder zu übertragen.

Über den zweiten Versorgungsanschluss können ausschließlich Daten, ausschließlich elektrische Energie oder aber beides übertragen werden, so dass die zweite elektrische und/oder elektronische Einrichtung mit den benötigten Informationen und Energien versorgbar ist.

Eine Weiterbildung der Erfindung sieht vor, dass ein Adapter mit dem ersten oder zweiten Versorgungsanschluss oder Steckeranschluss verbindbar ausgestaltet ist und der Adapter einen Eingangsanschluss für unterschiedliche Ladestationen aufweist. Über einen solchen Adapter besteht die Möglichkeit, unterschiedliche Energiequellen zu verwenden, um beispielsweise Energiespeicher in der orthopädietechnischen Komponente zu versorgen. Über den Adapter können Energiequellen mit unterschiedlicher Spannungsversorgung an die orthopädietechnische Komponente angeschlossen werden, um eine Versorgung mit Energie weitgehend unabhängig von dem Aufenthaltsort zu gewährleisten. Über den Adapter können Anschlüsse zu 12 V-Versorgungen, beispielsweise über einen Zigarettenanzünderanschluss, zu 5 V-Anschlüssen, zu USB-Anschlüssen, zu Anschlüssen von Batteriepacks oder zu einem Computer oder einem Notebook hergestellt werden. Über den Adapter können unterschiedliche Spannungspegel verarbeitet und auf den Datenleitungen und/oder Energieleitungen weitergeleitet werden. Ganz allgemein ist es über den Adapter möglich, unterschiedliche Kommunikationsprotokolle zu verwenden.

Eine Weiterbildung der Erfindung sieht vor, dass der Versorgungsanschluss und/oder Steckeranschluss eine Befestigungseinrichtung zur Festlegung an der jeweiligen elektrischen und/oder elektronischen Komponente aufweist. Die Befestigungseinrichtung kann als formschlüssige Festlegung und/oder kraftschlüssige Einrichtung ausgebildet sein. Damit ist es möglich, dass der Versorgungsanschluss als solcher veränderbar oder aber auch nur individuell positionierbar an der Komponente festgelegt werden kann. Formschlüssige Befestigungseinrichtungen können als Klettverschlüsse, Clipse oder Gewinde ausgebildet sein. An dem Versorgunganschluss kann eine Gewindestange oder eine Gewindebuchse angeordnet sein, die dann über eine Schraubverbindung mit einer korrespondierenden Mutter oder Schraube formschlüssig an der Komponente festlegbar ist. Alternativ oder ergänzende kann über einen Klettverschluss, eine Klebeschicht oder auch ein Magnet der Versorgungsanschluss bevorzugt reversibel an einer für den Patienten oder Orthopädietechniker günstigen Stelle der Komponente positioniert werden. Zwischen dem Versorgungsanschluss und der eigentlichen elektrischen oder elektronischen Einrichtung ist bevorzugt eine Kabelverbindung vorgesehen, die in oder an der Komponente verlegt wird und gegebenenfalls über eine Abdeckung gegen äußere Einflüsse abgeschirmt wird.

Eine weitere Ausgestaltung der Erfindung sieht vor, dass eine Steuerungs- oder Verteileinrichtung mit mehreren elektrischen und/oder elektronischen Komponenten mit Speichern elektrischer Energie gekoppelt ist, wobei die Steuerungs- oder Verteileinrichtung die Ladezustände der jeweiligen Speicher der elektrischen Energie erfasst und in Abhängigkeit der jeweiligen Ladezustände der Speicher einen Ladestrom von dem Versorgungsanschluss zu den Speichern verteilt oder elektrische Energie unter den Speichern verteilt, so dass eine möglichst gleichmäßige Ausnutzung der Speicherkapazität der elektrischen Speicher vorgenommen wird. Dadurch ist es möglich, dass zwischen den Komponenten und den einzelnen elektrischen oder elektronischen Einrichtungen ein Energieaustausch stattfindet oder in Abhängigkeit von dem jeweiligen Ladezustand der Speicher eine entsprechende Energiemenge oder Ladestrom von der Steuerungs- oder Verteileinrichtung dem jeweiligen Speicher zugeordnet wird. Ist der Energiespeicher leer oder weist im Vergleich mit den übrigen Energiespeichern den niedrigsten Ladezustand auf, wird dieser Energiespeicher bevorzugt mit Ladestrom versehen, da anzunehmen ist, dass die diesem Speicher zugeordnete elektrische oder elektronische Einrichtungen besonders häuft genutzt wird oder einen hohen Energieverbrauch aufweist und daher bevorzugt mit zusätzlicher Energie versorgt werden muss. Ebenso ist es möglich, über die Steuerungs- oder Verteileinrichtung einen Energieausgleich innerhalb der orthopädietechnischen Komponenten oder innerhalb des Systems auszuführen, so dass die Kapazität aller Energiespeicher in dem System der orthopädietechnischen Komponenten möglichst gleichmäßig ausgenutzt werden können. Dazu werden Unterschiede in den Ladezuständen der jeweiligen Speicher analysiert und bei einem hohen Ladezustand die Energie zu einem Speicher mit einem geringeren Ladezustand verteilt.

Die elektrischen und/oder elektronischen Einrichtungen sind elektrisch und/oder Daten übertragend miteinander gekoppelt, um über einen zentralen Anschluss alle elektrischen und/oder elektrischen Einrichtungen sowohl hinsichtlich der Energieversorgung als auch hinsichtlich eines Datenaustausches oder eines Datenabgleiches zu erreichen. Darüber hinaus erfolgt über die elektrische und/oder elektronische Kopplung eine Identifikation oder eine Identifizierbarkeit der einzelnen elektrischen und/oder elektronischen Einrichtungen, so dass ein individuell zusammengestelltes orthopädietechnisches System, wie beispielsweise eine Orthese oder Prothese, mit mehreren Komponenten von der jeweiligen Ladestation in ihrer Gesamtheit erfasst und hinsichtlich des Ladevorganges und/oder der Versorgung mit Daten individuell angesprochen werden kann.

Alle elektrischen und/oder elektronischen Einrichtungen sind in einer Weiterbildung der Erfindung mit einer Steuerungseinrichtung verbunden, die alle elektrischen und/oder elektronischen Einrichtungen hinsichtlich ihrer Funktion, ihres Aufbaus und ihres Typs identifiziert und deren Aktvierung und Deaktivierung von der Steuerungseinrichtung veranlasst wird. Durch die automatische Identifizierung durch die Steuerungseinrichtung wird die Ladestation mit allen notwendigen Informationen hinsichtlich der bereitzustellenden Energiemenge und der Art des Umfanges der bereitzustellenden Daten oder auch der Abfragemodalitäten der innerhalb der orthopädietechnischen Komponente aufgenommenen Daten versorgt.

Das erfindungsgemäße Verfahren zur Steuerung eines oben beschriebenen Systems sieht vor, dass alle in dem System vorhandenen und miteinander gekoppelten elektrischen und/oder elektronischen Einrichtungen von einer Steuerungseinrichtung angetriggert werden. Durch das Antriggern wird jede elektrische und/oder elektronische Einrichtung angesprochen oder über eine Datenabfrage zu einer Reaktion bewegt, so dass der Status und der Funktionsumfang der jeweiligen elektrischen und/oder elektronischen Einrichtung abgefragt werden. Nach der Abfrage werden Energie und/oder Datensignale an die jeweilige elektrische und/oder elektronische Einrichtung übermittelt, so dass ein an die jeweilige Komponente angepasste oder darauf zugeschnittene Daten- und Energieversorgung erfolgen kann.

Eine Weiterbildung des Verfahrens sieht vor, dass die Ladezustände mehrere elektrische Energiespeicher erfasst und während eines Ladevorganges die Ladeenergie auf der Grundlage der erfassten Ladezustände verteilt wird. Insbesondere werden Energiespeicher mit einer hohen Ladekapazität und/oder Energiespeicher mit einem geringen Ladezustand bevorzugt mit elektrischer Energie versorgt. Die bevorzugten oder besonders wichtigen oder großen Speicher können über einen längeren Zeitraum oder mit einem höheren Ladestrom versorgt, um das Gesamtsystem mit einer möglichst hohen Energiemenge versorgen zu können.

Mehrere elektrische Energiespeicher können zu einem Verbund zusammengefasst werden, so dass alle Energiespeicher alle elektrischen und/oder elektronischen Einrichtungen mit Energie versorgen können. Die Versorgung der elektrischen und/oder elektronischen Einrichtungen aus dem Verbund wird zentral von der Steuerungseinrichtung verteilt, so dass gewährleistet wird, dass alle elektrischen und/oder elektronischen Komponenten über eine maximale Laufzeit mit einer ausreichenden Menge an elektrischer Energie versorgt werden können. Darüber hinaus kann eine Priorisierung der jeweiligen elektrischen und/oder elektronischen Komponenten erfolgen. Sofern einige elektrische und/oder elektronische Komponenten nicht für die Funktionsweise des orthopädietechnischen Systems wesentlich sind, können diese im Falle eines kritischen niedrigen Energieniveaus abgeschaltet oder in ihrem Funktionsumfang reduziert werden.

Eine Weiterbildung sieht vor, dass derjenige elektrische Energiespeicher, der den höchsten Ladezustand aufweist, zuerst einem Verbraucher zugeordnet wird. Diese Zuordnung kann unabhängig von der örtlichen Zuordnung des jeweiligen Energiespeichers an einer orthopädietechnischen Komponente des Systems erfolgen. Beispielsweise kann ein Energiespeicher an einer Oberschenkelschiene, der eigentlich zur Versorgung eines Antriebes eines orthetischen Kniegelenkes vorgesehen ist, zur Aktuierung eines Orthesenknöchelgelenkes verwendet werden, wenn der Energiespeicher an der Oberschenkelschiene eine höhere Energiemenge zur Verfügung hat als der eigentlich dem Knöchelantrieb zugeordnete Energiespeicher.

Die Energiespeicher können einen Ladungsaustauch untereinander durchführen, um eine gleichmäßige Auslastung der Energiespeicher zu gewährleisten.

Die Funktion der elektrischen und/oder elektronischen Einrichtungen kann aufgrund der gekoppelten Komponenten jeweils individuell aktiviert oder deaktiviert werden. Jede Komponente hat einen Funktionsumfang, der der jeweiligen Komponente zugeordnet und von der Steuerungseinrichtung abfragbar ist. Einige Funktionen können in Kombination mit anderen orthopädietechnischen Komponenten kontraproduktiv sein oder nicht benötigt werden, so dass eine automatische Abschaltung oder Aktivierung einzelner Funktionen in Abhängigkeit von dem Funktionsumfang und dem sich aus der Kombination der einzelnen orthopädietechnischen Komponenten ergebenden Indikation vorgenommen werden kann.

Eine Weiterbildung der Erfindung sieht vor, dass die elektrischen und/oder elektronischen Einrichtungen nach der Kopplung der orthopädietechnischen Komponenten authentifiziert werden und dass der Funktionsumfang der elektrischen und/oder elektronischen Einrichtungen und/oder eine Energiefreigabe zu diesen Einrichtungen in Abhängigkeit der miteinander kombinierten elektrischen und/oder elektronischen Einrichtungen festgelegt wird. Durch eine solche Authentifizierung wird verhindert, dass Funktionen ermöglicht werden, die für das Gesamtsystem schädlich wären.

Nachfolgend werden Ausführungsbeispiele der Erfindung anhand der beigefügten Figuren näher erläutert. Es zeigen:
- Figur 1 -: eine erste Variante des Systems; sowie
- Figur 2 -: eine zweite Variante.

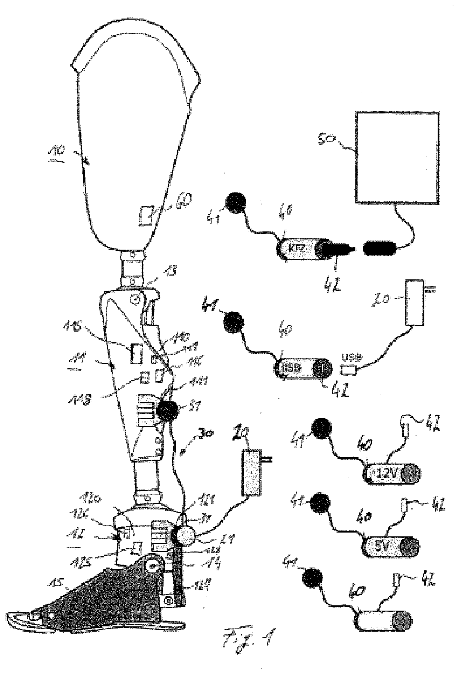

In der Figur 1 ist in einer schematischen Darstellung eine Prothese einer unteren Extremität mit mehreren orthopädietechnischen Komponenten 10, 11, 12, 15 dargestellt. Als erste orthopädietechnische Komponente 10 ist ein Oberschenkelschaft mit einem Anschlussadapter zur Befestigung an einem Oberteil eines Prothesenkniegelenkes gezeigt. An dem Prothesenkniegelenk ist eine zweite orthopädietechnische Komponente 11 schwenkbar um eine Schwenkachse 13 in Gestalt eines Unterschenkelteils angeordnet. An dem Unterschenkelteil als zweite orthopädietechnische Komponente 11 ist eine dritte orthopädietechnische Komponente 12 in Gestalt eines prothetischen Knöchelgelenks befestigt, an dem als vierte orthopädietechnische Komponente ein Prothesenfuß 15 schwenkbar um eine Schwenkachse 14 gelagert ist. Der Oberschenkelschaft als erste orthopädietechnische Komponente 10 ist eine reine passive Komponente und dient zur Aufnahme eines Oberschenkelstumpfes, um das Prothesenbein sicher an dem Patienten festlegen zu können. Über den Oberschenkelschaft und einen darin angeordneten Prothesenliner kann beispielsweise über ein Unterdrucksystem eine mechanische Festlegung des Prothesenbeines an dem Patienten erfolgen. Alternativ zu einer mehrteiligen Prothese kann das System als eine Orthese ausgebildet sein, ebenso kann als orthopädietechnische Komponente ein Rollstuhl oder eine prothetische oder orthetische Einrichtung für eine obere Extremität eingesetzt werden, beispielsweise ein Prothesenarm, eine Armorthese oder eine Schulterorthese. Als System werden auch Kombinationen von Prothesen, Orthesen und Rollstühlen oder anderen Mobilitätshilfen angesehen.

In dem dargestellten System ist in der zweiten orthopädietechnischen Komponente 11 eine erste elektrische und/oder elektronische Einrichtung 110 in Gestalt eines Hydraulikaktuators angeordnet. Der Hydraulikaktuator kann als passive Komponente ausgebildet sein und einen Hydraulikdämpfer aufweisen, der Stellantriebe aufweist, über die Ventile geöffnet oder geschlossen werden, um einen Extensionswiderstand und/oder Flexionswiderstand einstellen zu können. An dem Hydraulikaktuator kann zudem ein Speicher 115 zur Speicherung elektrischer Energie angeordnet oder diesem zugeordnet sein, um die Stellantriebe mit Energie zu versorgen. Bei einer Ausgestaltung der elektrischen und/oder elektronischen Einrichtung 110 als ein aktiver Aktuator ist eine Pumpeinrichtung oder ein mechanischer Energiespeicher, z.B. eine Feder oder Druckspeicher, dem Hydraulikdämpfer zugeordnet, über den es möglich ist, eine Bewegung des Oberschenkelschaftes relativ zu dem Unterschenkel zu bewirken oder zu unterstützen. Dazu wird die Energie aus dem Energiespeicher 115 in Bewegungsenergie umgewandelt, so dass beispielsweise eine Kolbenstange aus dem Hydraulikaktuator hinausbewegt wird, um eine Extensionsbewegung zu unterstützen oder zu bewirken. Umgekehrt kann durch eine Pumpe oder einen Druckspeicher Hydraulikfluid umgepumpt werden, so dass eine Kolbenstange in ein Gehäuse des Hydraulikaktuators eingefahren wird, damit sich der Abstand zwischen zwei Befestigungspunkten des Hydraulikaktuators an dem Oberteil und dem Unterteil des Prothesengelenkes verkürzt, um eine Flexionsbewegung auszuführen.

Distal zu der ersten elektrischen und/oder elektronischen Komponente 110 ist die zweite elektrische und/oder elektronische Komponente 120 in der orthopädietechnischen Komponente 12 in Gestalt eines Prothesenknöchelgelenkes angeordnet. Innerhalb des Prothesenknöchelgelenkes kann ebenfalls ein elektrischer und/oder hydraulischer Aktuator angeordnet sein, der über einen Speicher 125 für elektrische Energie mit elektrischer Energie versorgbar ist. Neben dem Speicher 125 für elektrische Energie ist in dem Prothesenknöchelgelenk eine Steuerungseinrichtung 126 angeordnet, an der Steuerungseinrichtung 116 in der ersten elektrischen und/oder elektronischen Einrichtung 110 angeordnet, um beispielsweise einen Antrieb 118, 128 zu aktivieren oder zu deaktivieren oder Daten eines Sensors 117, 127 zu verarbeiten, Sensordaten zu speichern und zur Steuerung weiterzuverwenden. In der Steuerungseinrichtung 116, 126 kann auch ein Datenspeicher sowie eine Verarbeitungsschaltung integriert sein.

Sowohl an der zweiten orthopädietechnischen Komponente 11 als auch an der dritten orthopädietechnischen Komponente 12 ist jeweils ein Versorgungsanschluss 111, 121 angeordnet, über den Energie und/oder Daten der jeweiligen elektrischen und/oder elektronischen Einrichtung 110, 120 zugeführt werden kann. Die Daten sowie die elektrische Energie können von einer Ladestation 20 auf den jeweiligen Versorgungsanschluss 111, 121 übertragen werden. Dazu ist an der Ladestation 20 ein Stecker 21 angeordnet, der mit dem jeweiligen Versorgungsanschluss 111, 121 kompatibel ist. In dem dargestellten Ausführungsbeispiel der Figur 1 wird der Stecker 21 der Ladestation 20 mit einem kaskadierenden Steckersystem einer Steckerverbindung 30 gekoppelt, die die beiden Versorgungsanschlüsse 111, 121 miteinander verbindet. An der Steckerverbindung 30 sind zwei Stecker 31 angeordnet, die auf einer Seite Kontakte aufweisen, die kompatibel zu den Kontakten der jeweiligen Versorgungsanschlüsse 111, 121 ausgebildet sind. Auf der den Versorgungsanschlüssen 111, 121 abgewandten Seite weisen die Stecker 31 Aufnahmen oder Buchsen auf, die mit den Kontakten des Steckers 21 der Ladestation 20 kompatibel sind.

Wird der Stecker 21 der Ladestation 20 auf die Rückseite eines Steckers 31 gesteckt, der über ein Kabel mit einem korrespondierenden Stecker 31 verbunden ist, so dass beide Versorgungsanschlüsse 111, 121 miteinander verbunden sind, können Energie und Daten von der Ladestation 20 sowohl auf die erste als auch auf die zweite elektronische und/oder elektrische Einrichtung 110, 120 übertragen werden. Dadurch werden die Speicher 115, 125 zur Speicherung elektrischer Energie aufgefüllt sowie die Steuerungseinrichtungen 116, 126 mit Daten wie Programmen, Steuerungsdaten, Softwareupdates oder dergleichen versorgt. Über das System aus Ladestation 20 mit Stecker 21, Versorgungsanschlüssen 111, 121 sowie die Steckerverbindung 30 mit den Steckern 31 ist es möglich, ein elektrisches Verbindungssystem für orthopädietechnische Komponenten 11, 12, insbesondere Orthesen, Prothesen und/oder Rollstühle, bereitzustellen, mit dem es möglich ist, die jeweiligen elektrischen und/oder elektronischen Einrichtungen 110, 120 aufzuladen, miteinander zu verbinden, um Energie aus den jeweiligen Energiespeichern 115, 125 untereinander zu verteilen oder um Steuerungsabläufe miteinander zu koordinieren. Über das System ist es möglich, nicht nur extern Daten aus der Ladestation 20 sowie elektrische Energie dem orthopädietechnischen System, beispielsweise der Orthese, Prothese oder dem Rollstuhl zuzuführen, sondern auch innerhalb der orthopädietechnischen Einrichtung zwischen den jeweiligen orthopädietechnischen Komponenten 11, 12 einen Energie- und/oder Datenaustausch zu ermöglichen.

Wenn in den unterschiedlichen Komponenten 11, 12 mit den verschiedenen elektrischen und/oder elektronischen Einrichtungen 110, 120 unterschiedliche maximal benötigte Leistungen in dem jeweiligen Verbraucher, beispielsweise Antrieb 118, 128, oder zum Laden der Speicher 115, 125 vorhanden sind, kann die jeweils maximal benötigte Leistung eines Verbrauchers 118, 128 oder einer Steuerungseinrichtung 116, 126 über einen elektrischen Widerstand kodiert werden. Durch die Kompatibilität der Versorgungsanschlüsse 111, 121 mit den jeweiligen Steckern 21, 31 ist es möglich, mehrere Ladeanschlüsse an einer orthopädietechnischen Komponente bereitzustellen, um es einen Patienten zu erleichtern, das orthopädietechnische Hilfsmittel mit einer Ladestation 20 zu koppeln. Dadurch kann der Nutzer den für ihn am besten zu erreichenden Versorgungsanschluss 111, 121 frei auswählen, so dass ein oder mehrere Anschlüsse zum zentralen Laden aller elektrischer und/oder elektronischer Einrichtungen 110, 120 vorhanden sind. Das Laden mit elektrischer Energie ebenso wie die Versorgung mit Daten kann gleichzeitig oder sequentiell erfolgen. Sind beispielsweise die elektrischen und/oder elektronischen Einrichtungen 110, 120 nicht über die Steckerverbindung 30 miteinander gekoppelt, können Daten und Energie über die Ladestation 20 nacheinander durch den Stecker 21 übertragen werden. Die jeweilige elektronische und/oder elektrische Einrichtung 110, 120 ist mit einer Kodierung versehen, so dass über die Ladestation 20 erkannt wird, welche Einrichtung gerade mit Energie und/oder Daten versorgt werden soll, so dass sowohl die richtige Energiemenge als auch die richtigen Daten übertragen werden.

Ist die Ladestation 20 nicht angeschlossen, kann über die Steckerverbindung 30 ein Daten- und Energieaustausch zwischen den Einrichtungen 110, 120 erfolgen. Ebenfalls ist es möglich, über eine Steckerverbindung 30 mehrere orthopädietechnische Hilfsmittel miteinander zu kombinieren, beispielsweise eine Orthese oder eine Prothese mit einem Rollstuhl, der beispielsweise über einen größeren Energiespeicher in Gestalt einer Batterie verfügt, so dass zur Aufrechterhaltung der Mobilität des Patienten Energie aus dem Rollstuhl in die Orthese oder Prothese übertragen werden kann.

In der Figur 1 sind weitere Ladestationen 20 und Energiespeicher dargestellt, die unterschiedliche Spannungen bereitstellen, beispielsweise eine 12V-Spannung, eine 5V-Spannung oder über einen speziellen Transformator oder einem herstellerspezifischen Ladegerät verschiedene Spannungen zur Verfügung stellen. Ebenfalls ist ein Energiespeicher über einen USB-Anschluss mit einem Netzteil einer Ladestation 20 koppelbar, so dass ein mobiler Speicher für elektrische Energie vorhanden ist. Ebenso kann ein Anschluss für einen Zigarettenanzünder als Energieversorgung genutzt werden, der beispielsweise auch mit einem externen Energiespeicher 50 gekoppelt werden kann, an dem ein entsprechender Adapter angeordnet ist. Der externe Energiespeicher 50, der auch ein Rechner oder tragbarer Computer sein kann, kann auch unabhängig von einem Zigarettenanzünder mit einer orthopädietechnischen Komponente gekoppelt werden. Über einen oder mehrere Adapter 40, die mit einem jeweiligen Stecker 41 versehen sind, die mit den jeweiligen Versorgungsanschlüssen 111, 121 kompatibel sind, ist es möglich, die unterschiedlichsten Energiequellen zu nutzen, um die jeweilige elektronische und/oder elektrische Einrichtung mit Energie zu versorgen. Die Adapter 40 können unterschiedliche Arten von Energiequellen mit den jeweiligen orthopädietechnischen Komponenten verbinden, wobei innerhalb der Adapter 40 unterschiedliche Spannungspegel den jeweiligen Einrichtungen zugeleitet werden können. Innerhalb der Adapter 40 kann der jeweils benötigte Spannungspegel eingerichtet werden, beispielsweise durch Erhöhung oder Verringerung der von dem jeweiligen Energiespeicher empfangenen Spannung. In jedem Adapter 40 ist ein Eingangsanschluss 42 für die jeweils verwendete Ladestation 20 oder den jeweils verwendeten Energiespeicher vorgesehen.

An der Prothese ist eine Steuerungseinrichtung 60 angeordnet, die auf drahtlose oder drahtgebundene Art und Weise alle elektrischen und/oder elektronischen Einrichtungen 110, 120 identifiziert und deren Aktivierung oder Deaktivierung veranlasst. In einer alternativen Ausgestaltung kann die Steuerungseinrichtung 60 in einer der Einrichtungen 110, 120 untergebracht sein und als Hauptsteuereinrichtung oder Master die übrigen angeschlossenen Komponenten identifizieren, aktivieren und deaktivieren. Dazu werden sämtliche elektrischen und/oder elektronischen Einrichtungen 110, 120 von der Steuerungseinrichtung 60 angetriggert, der jeweils vorgesehene Status und Funktionsumfang abgefragt und Energie und/oder Datensignale an die jeweilige elektrische und/oder elektronische Einrichtung 110, 120 übermittelt.

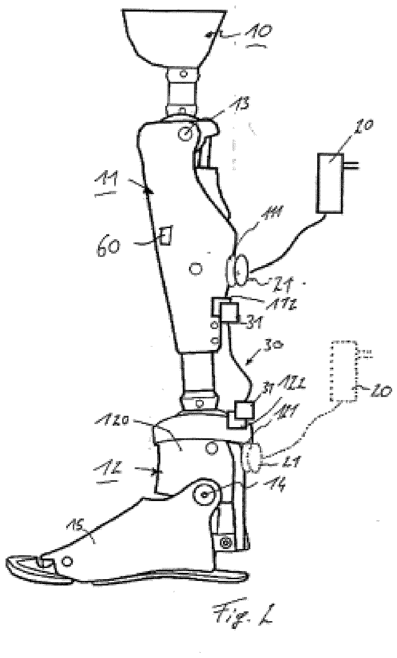

In der Figur 2 ist eine Variante der Erfindung dargestellt, bei der statt einer überbrückenden Kopplung der beiden Versorgungsanschlüsse 111, 121 über die Steckerverbindung 30 zur Weiterleitung von Energie und Daten sowie zur Durchleitung von Energie und Daten von der Ladestation 20 über die jeweiligen Stecker 31 separate Steckeranschlüsse 112, 122 an den beiden elektrischen und/oder elektronischen Einrichtungen 110 , 120 angeordnet sind. Diese Steckeranschlüsse 112, 122 sind zusätzlich zu den Versorgungsanschlüssen 111, 121 vorgesehen. In der Figur 2 ist der zweite Versorgungsanschluss 121 an der zweiten orthopädietechnischen Komponente 12 als zusätzlicher oder optionaler Versorgungsanschluss vorgesehen. Es besteht auch die Möglichkeit, dass auf den zweiten Versorgungsanschluss 121 verzichtet werden kann. Daher die zweite Ladestation 20 nur gestrichelt dargestellt. Bei einer Ausstattung mehrerer oder aller orthopädietechnischen Komponenten, die eine elektrische und/oder elektronische Einrichtung aufweisen, mit Versorgungsanschlüssen besteht der Vorteil darin, dass die Ladezeiten verkürzt werden können und dass individuell und schneller Energie und Daten an die jeweilige elektrische und/oder elektronische Einrichtung übertragen werden kann. Die Kopplung mit einer externen Einrichtung, also mit einer Ladestation 20, kann an mehreren Versorgungsanschlüssen 111, 121 erfolgen. Getrennt von den Versorgungsanschlüssen 111, 121 sind die Steckeranschlüsse 112, 122 an der jeweiligen orthopädietechnischen Komponente 11, 12 angeordnet, über die ein Datenaustausch und/oder Energieaustausch zwischen den einzelnen elektrischen und/oder elektronischen Einrichtungen 110, 120 verwirklicht wird. Die Stecker 31 der Steckerverbindung 30 können unterschiedlich von den Steckern 21 der Ladestationen 20 ausgebildet oder mit anderen Kontakten belegt sein, alternativ dazu sind die Steckeranschlüsse 112, 122 mit den Versorgungsanschlüssen 111, 121 und damit auch mit den Steckern 21 der Ladestationen 20 koppelbar und kompatibel. Die Energieversorgung und gegebenenfalls Versorgung mit Daten erfolgt über die Versorgungsanschlüsse 111, 121, während die Verbindung zwischen den elektrischen und/oder elektronischen Komponenten 110, 120 über die Steckerverbindung 30 erfolgt.

Sollte eine weitere Komponente intern verbunden werden, würde ein dritter Stecker 31 an der Steckerverbindung 30 angeordnet werden, bei weiteren anzuschließenden Komponenten weitere Stecker.

Um das System aus einer Vielzahl elektrischer und/oder elektronischer Komponenten 110, 120 einrichten und steuern zu können, werden alle in dem System vorhandenen und miteinander gekoppelten elektrischen und/oder elektronischen Einrichtungen 110, 120 von der Steuerungseinrichtung 60 angetriggert, beispielsweise per Funk, oder, wenn die Steuerungseinrichtung 60 an einer orthopädietechnischen Komponente mit einer elektrischen und/oder elektronischen Einrichtung verbunden ist, per Kabel, wobei die anderen elektrischen und/oder elektronischen Komponenten bevorzugt über die Steckerverbindung 30, die auch innerhalb der orthopädietechnischen Komponente oder Komponenten verlegt sein kann, angetriggert werden. Die Steuereinrichtung 60 fragt den Status der jeweiligen Einrichtung, also ob sie aktiviert ist, welchen Softwarestand sie hat, wie der Ladezustand ist und welchen Funktionsumfang der jeweiligen Einrichtung zugeordnet ist. Auf Basis der empfangenen Informationen über Status und Funktionsumfang werden Energie und/oder Datensignale an die jeweilige Einrichtung übermittelt. Die Steuerungseinrichtung 60 kann dazu mit der jeweiligen Ladestation 20 gekoppelt sein, beispielsweise drahtlos oder über eine Drahtverbindung. Ebenfalls ist es möglich, dass die Steuerungseinrichtung 60 Teil der Ladestation 20 ist oder mit dem Versorgungsanschluss 111, 121 gekoppelt ist, über den Energie und/oder Daten von außen in das System hineingebracht werden.

Die Steuerungseinrichtung 60, die in dem Ausführungsbeispiel der Figur 2 in der zweiten orthopädietechnischen Komponente 11 angeordnet ist, um auf eine gelenkübergreifende Kabelverbindung verzichten zu können, ist mit allen Energiespeichern gekoppelt und überwacht, welches Energieniveau der jeweilige Energiespeicher hat. Auf Basis der Wichtigkeit des Energiespeichers für die Funktionalität des Gesamtsystems kann die Ladeenergie mit unterschiedlichen Prioritäten verteilt werden. Die für die Funktionsfähigkeit unbedingt notwendigen elektrischen und/oder elektronischen Einrichtungen werden bevorzugt mit elektrischer Energie versorgt, während Zusatzeinheiten, die einen erhöhten Funktionsumfang und gegebenenfalls einen erhöhten Komfort gewährleisten, später oder mit einem geringeren Anteil an Energie versorgt werden. Während des Ladevorganges kann über die Steuereinrichtung 60 die Energieverteilung verändert werden. Ist die Zufuhr externer Energie abgeschlossen, wird über die Steuerungseinrichtung 60 die interne Energieverteilung zwischen den einzelnen Energiespeichern 115, 125 innerhalb des Systems gesteuert. So kann der Energiespeicher mit dem aktuell höchsten Ladezustand zuerst einem Verbraucher zugeordnet werden, alternativ oder ergänzend kann ein Ladungsaustauch oder Energieaustausch zwischen den Energiespeichern erfolgen, um entweder eine gleichmäßige Energieverteilung zu erzielen oder aber, um einen priorisierten Verbraucher mit dem jeweils zugeordneten Energiespeicher mit einer erhöhten Energiemenge zu versorgen.

Aufgrund der durch das Antriggern erhaltenen Informationen über den Status und die Art der elektrischen und/oder elektronischen Komponente ist es möglich, dass bei bestimmten Kombinationen unterschiedlicher elektrischer oder elektronsicher Einrichtungen bestimmte Funktionen nicht gewünscht sind oder wegfallen müssen, so dass in Abhängigkeit von der jeweils detektierten elektrischen und/oder elektronischen Einrichtung an der jeweiligen orthopädietechnischen Komponente eine entsprechende Funktion aktiviert oder deaktiviert wird.

## Patentansprüche

1. System aus mehreren orthopädietechnischen Komponenten (10, 11, 12, 15), die miteinander gekoppelt sind, mit einer ersten elektronischen und/oder elektrischen Einrichtung (110), die einen ersten Versorgungsanschluss (111) aufweist, über den die erste elektrische und/oder elektronische Einrichtung (110) mit Energie und/oder Daten von einer Ladestation (20) über einen Stecker (21) versorgbar ist, **dadurch gekennzeichnet, dass** zumindest eine zweite elektrische und/oder elektronische Einrichtung (120) an einer der Komponenten (12) angeordnet ist, die einen separaten zweiten Versorgungsanschluss (121) und/oder einen Steckeranschluss (122) aufweist, der oder die mit der ersten elektronischen und/oder elektrischen Einrichtung (110) zur Übertragung von Energie und/oder Daten über den ersten Versorgungsanschluss (111) oder einen separaten Steckeranschluss (112) koppelbar ist oder sind.

2. System nach Anspruch 1, **dadurch gekennzeichnet, dass** die erste und zweite elektronische und/oder elektrische Einrichtung (110, 120) über eine Steckerverbindung (30) zwischen den Steckeranschlüssen (112, 122), den Versorgungsanschlüssen (111, 121) oder einem Steckeranschluss (112, 122) und einem Versorgungsanschluss (111, 121) miteinander koppelbar sind.

3. System nach einem der voranstehenden Ansprüche, **dadurch gekennzeichnet, dass** die Versorgungsanschlüsse (111, 121) der ersten und zweiten elektronischen und/oder elektrischen Einrichtungen (110, 120) mit dem Steckeranschluss (112, 122) kompatibel sind.

4. System nach einem der voranstehenden Ansprüche, **dadurch gekennzeichnet, dass** der Versorgungsanschluss (111) der ersten elektronischen und/oder elektrischen Einrichtung (110) mit dem Steckeranschluss (122) zumindest der zweiten elektrischen und/oder elektronischen Einrichtung (120) kompatibel ist.

5. System nach einem der voranstehenden Ansprüche, **dadurch gekennzeichnet, dass** die elektrische und/oder elektronische Einrichtung (110, 120) einen Aktuator (118, 128), eine Steuerungseinrichtung (116, 126), eine Verarbeitungsschaltung, einen Sensor (117, 127), einen Datenspeicher, ein hydraulischen Dämpfer und/oder einen Energiespeicher (115, 125) aufweist.

6. System nach einem der voranstehenden Ansprüche, **dadurch gekennzeichnet, dass** die orthopädietechnischen Komponenten (10, 11, 12) mechanisch und/oder elektrisch miteinander gekoppelt sind.

7. System nach einem der voranstehenden Ansprüche, **dadurch gekennzeichnet, dass** über den Steckeranschluss (112, 122) ausschließlich Daten, ausschließlich elektrische Energie oder beides übertragbar sind.

8. System nach einem der voranstehenden Ansprüche, **dadurch gekennzeichnet, dass** ein Adapter (40) mit dem ersten oder zweiten Versorgungsanschluss (111, 121) oder Steckeranschluss (112, 122) verbindbar ausgestaltet ist und der Adapter (40) einen Eingangsanschluss (42) für unterschiedliche Ladestationen (20) aufweist.

9. System nach einem der voranstehenden Ansprüche, **dadurch gekennzeichnet, dass** der Versorgungsanschluss (111, 121) und/oder Steckeranschluss (112, 122) eine Befestigungseinrichtung zur Festlegung an der jeweiligen Komponente (11, 12) aufweist.

10. System nach einem der voranstehenden Ansprüche, **dadurch gekennzeichnet, dass** eine Steuerungseinrichtung (60) mit mehreren elektrischen oder elektronischen Einrichtungen (110, 120) mit Speichern (115, 125) für elektrische Energie gekoppelt ist, die Steuerungseinrichtung (60) die Ladezustände der Speicher (115, 125) erfasst und in Abhängigkeit der Ladezustände der Speicher (115, 125) Ladestrom von dem Versorgungsanschluss (111, 121) zu den Speichern (115, 125) oder elektrische Energie unter der Speichern (115, 125) verteilt.

11. System nach einem der voranstehenden Ansprüche, **dadurch gekennzeichnet**, dassdie elektrischen und/oder elektronischen Einrichtungen (110, 120) elektrisch und/oder datenübertragend miteinander gekoppelt sind.

12. System nach Anspruch 11, **dadurch gekennzeichnet, dass** alle elektrischen und/oder elektronischen Einrichtungen (110, 120) mit einer Steuerungseinrichtung (60) verbunden sind, die Steuerungseinrichtung (60) alle elektrischen und/oder elektronischen Einrichtungen (110, 120) identifiziert und deren Aktivierung oder Deaktivierung veranlasst.

13. Verfahren zur Steuerung eines Systems nach einem der voranstehenden Ansprüche, **dadurch gekennzeichnet, dass** alle in dem System vorhandenen und miteinander gekoppelten elektrischen und/oder elektronischen Einrichtungen (110, 120) von einer Steuerungseinrichtung (60) angetriggert werden, deren Status und Funktionsumfang abgefragt werden und Energie und/oder Datensignale an die jeweiligen elektrischen und/oder elektronischen Einrichtungen (110, 120) übermittelt werden.

14. Verfahren nach Anspruch 13, **dadurch gekennzeichnet, dass** Ladezustände mehrerer Speicher (115, 125) elektrischer Energie erfasst und während eines Ladevorganges die Ladeenergie auf der Grundlage der erfassten Ladezustände verteilt wird.

15. Verfahren nach Anspruch 13 oder 14, **dadurch gekennzeichnet, dass** mehrere Speicher (115, 125) elektrischer Energie zu einem Verbund zusammengefasst und die Versorgung der elektrischen und/oder elektronischen Einrichtungen (110, 120) aus dem Verbund zentral von der Steuerungseinrichtung (60) verteilt wird.

16. Verfahren nach Anspruch 15, **dadurch gekennzeichnet, dass** der Speicher (115, 125) elektrischer Energie mit dem höchsten Ladezustand zuerst einem Verbraucher zugeordnet wird.

17. Verfahren nach einem der Ansprüche 13 bis 16, **dadurch gekennzeichnet, dass** Speicher (115, 125) elektrischer Energie einen Ladungsaustausch untereinander durchführen.

18. Verfahren nach einem der Ansprüche 13 bis 17, **dadurch gekennzeichnet, dass** Funktionen der elektrischen und/oder elektronischen Einrichtungen (110, 120) aufgrund der gekoppelten Komponenten (10, 11, 12) aktiviert oder deaktiviert werden.

19. Verfahren nach einem der Ansprüche 13 bis 18, **dadurch gekennzeichnet, dass** die elektrischen und/oder elektronischen Einrichtungen (110, 120) nach der Koppelung der Komponenten (10, 11, 12) authentifiziert werden und deren Funktionsumfang und/oder Energiefreigabe in Abhängigkeit der miteinander kombinierten elektrischen und/oder elektronischen Einrichtungen (110, 120) festgelegt wird.

## Claims

1. A system made up of multiple orthopedic components (10, 11, 12, 15) which are coupled to one another, having a first electronic and/or electrical unit (110), which comprises a first supply terminal (111), via which the first electrical and/or electronic unit (110) can be supplied with energy and/or data from a charging station (20) via a plug (21), **characterized in that** at least one second electrical and/or electronic unit (120) is arranged on one of the components (12), which comprises a separate supply terminal (121) and/or a plug terminal (122), which can be coupled to the first electronic and/or electrical unit (110) to transmit energy and/or data via the first supply terminal (111) or a separate plug terminal (112).

2. The system as claimed in claim 1, **characterized in that** the first and second electronic and/or electrical unit (110, 120) can be coupled to one another via a plug connection (30) between the plug terminals (112, 122), the supply terminals (111, 121), or one plug terminal (112, 122) and one supply terminal (111, 121).

3. The system as claimed in any one of the preceding claims, **characterized in that** the supply terminals (111, 121) of the first and second electronic and/or electrical units (110, 120) are compatible with the plug terminal (112, 122).

4. The system as claimed in any one of the preceding claims, **characterized in that** the supply terminal (111) of the first electronic and/or electrical unit (112) is compatible with the plug terminal (122) of at least the second electrical and/or electronic unit (120).

5. The system as claimed in any one of the preceding claims, **characterized in that** the electrical and/or electronic unit (110, 120) comprises an actuator (118, 128), a control unit (116, 126), a processing circuit, a sensor (117, 127), a data memory, a hydraulic damper, and/or an energy accumulator (115, 125).

6. The system as claimed in any one of the preceding claims, **characterized in that** the orthopedic components (10, 11, 12) are mechanically and/or electrically coupled to one another.

7. The system as claimed in any one of the preceding claims, **characterized in that** exclusively data, exclusively electric energy, or both are transmittable via the plug terminal (112, 122).

8. The system as claimed in any one of the preceding claims, **characterized in that** an adapter (40) is designed to be connectable to the first or second supply terminal (111, 121) or plug terminal (112, 122) and the adapter (40) comprises an input terminal (42) for different charging stations (20).

9. The system as claimed in any one of the preceding claims, **characterized in that** the supply terminal (111, 121) and/or plug terminal (112, 122) comprises a fastening unit for the fixation on the respective component (11, 12).

10. The system as claimed in any one of the preceding claims, **characterized in that** a control unit (60) having multiple electrical and/or electronic units (110, 120) is coupled to accumulators (115, 125) for electric energy, the control unit (60) detects the charge levels of the accumulators (115, 125) and distributes charging current from the supply terminal (111, 121) to the accumulators (115, 125) or electric energy among the accumulators (115, 125) in dependence on the charge levels of the accumulators (115, 125).

11. The system as claimed in any one of the preceding claims, **characterized in that** the electrical and/or electronic units (110, 120) are coupled to one another electrically and/or to transmit data.

12. The system as claimed in claim 11, **characterized in that** all electrical and/or electronic units (110, 120) are connected to a control unit (60), the control unit (60) identifies all electrical and/or electronic units (110, 120) and prompts the activation or deactivation thereof.

13. A method for controlling a system as claimed in any one of the preceding claims, **characterized in that** all electrical and/or electronic units (110, 120) which are provided in the system and are coupled to one another are triggered by a control unit (60), the status and functional scope thereof are queried, and energy and/or data signals are transmitted to the respective electrical and/or electronic units (110, 120).

14. The method as claimed in claim 13, **characterized in that** charge levels of multiple accumulators (115, 125) of electric energy are detected and the charging energy is distributed on the basis of the detected charge levels during a charging procedure.

15. The method as claimed in claim 13 or 14, **characterized in that** multiple accumulators (115, 125) of electric energy are combined to form a composite and the supply of the electrical and/or electronic units (110, 120) from the composite is distributed centrally by the control unit (60).

16. The method as claimed in claim 15, **characterized in that** the accumulator (115, 125) of electric energy having the highest charge level is first assigned to a consumer.

17. The method as claimed in any one of claims 13 to 16, **characterized in that** accumulators (115, 125) of electric energy carry out a charge exchange with one another.

18. The method as claimed in any one of claims 13 to 17, **characterized in that** functions of the electrical and/or electronic units (110, 120) are activated or deactivated due to the coupled components (10, 11, 12).

19. The method as claimed in any one of claims 13 to 18, **characterized in that** the electrical and/or electronic units (110, 120) are authenticated after the coupling of the components (10, 11, 12) and the functional scope and/or energy release thereof is established in dependence on the electrical and/or electronic units (110, 120) combined with one another.

## Revendications

1. Système constitué de plusieurs composants orthopédiques (10, 11, 12, 15) couplés les uns aux autres, comportant un premier dispositif électronique et/ou électrique (110) qui présente une première borne d'alimentation (111) par laquelle le premier dispositif électrique et/ou électronique (110) peut être alimenté en énergie et/ou en données à partir d'une station de charge (20) par le biais d'une fiche (21), **caractérisé en ce que en ce qu'**au moins un deuxième dispositif électrique et/ou électronique (120) est disposé sur l'un des composants (12) et présente une deuxième borne d'alimentation séparée (121) et/ou une borne à fiche (122) qui peut/peuvent être couplée(s) au premier dispositif électronique et/ou électrique (110) pour la transmission d'énergie et/ou de données via la première borne d'alimentation (111) ou via une borne à fiche séparée (112).

2. Système selon la revendication 1, **caractérisé en ce que** le premier et le deuxième dispositif électronique et/ou électrique (110, 120) peuvent être couplés l'un à l'autre via une connexion à fiche (30) entre les bornes à fiche (112, 122), les bornes d'alimentation (111, 121) ou une borne à fiche (112, 122) et une borne d'alimentation (111, 121).

3. Système selon l'une des revendications précédentes, **caractérisé en ce que** les bornes d'alimentation (111, 121) des premier et deuxième dispositifs électroniques et/ou électriques (110, 120) sont compatibles avec la borne à fiche (112, 122).

4. Système selon l'une des revendications précédentes, **caractérisé en ce que** la borne d'alimentation (111) du premier dispositif électronique et/ou électrique (110) est compatible avec la borne à fiche (122) au moins du deuxième dispositif électrique et/ou électronique (120).

5. Système selon l'une des revendications précédentes, **caractérisé en ce que** le dispositif électrique et/ou électronique (110, 120) comprend un actionneur (118, 128), un dispositif de commande (116, 126), un circuit de traitement, un capteur (117, 127), un moyen de stockage de données, un amortisseur hydraulique et/ou un moyen de stockage d'énergie (115, 125).

6. Système selon l'une des revendications précédentes, **caractérisé en ce que** les composants orthopédiques (10, 11, 12) sont couplés mécaniquement et/ou électriquement les uns aux autres.

7. Système selon l'une des revendications précédentes, **caractérisé en ce qu'**exclusivement des données, exclusivement de l'énergie électrique ou les deux peuvent être transmises par la borne à fiche (112, 122).

8. Système selon l'une des revendications précédentes, **caractérisé en ce qu'**un adaptateur (40) est conçu de manière à pouvoir être connecté à la première ou à la deuxième borne d'alimentation (111, 121) ou à la borne à fiche (112, 122), et l'adaptateur (40) présente une borne d'entrée (42) pour différentes stations de charge (20).

9. Système selon l'une des revendications précédentes, **caractérisé en ce que** la borne d'alimentation (111, 121) et/ou la borne à fiche (112, 122) présente un dispositif de fixation au composant respectif (11, 12).

10. Système selon l'une des revendications précédentes, **caractérisé en ce qu'**un dispositif de commande (60) est couplé à plusieurs dispositifs électriques ou électroniques (110, 120) ayant des moyens de stockage (115, 125) pour l'énergie électrique, le dispositif de commande (60) détecte les états de charge des moyens de stockage (115, 125) et, en fonction des états de charge des moyens de stockage (115, 125), distribue un courant de charge depuis la borne d'alimentation (111, 121) vers les moyens de stockage (115, 125) ou distribue de l'énergie électrique entre les moyens de stockage (115, 125).

11. Système selon l'une des revendications précédentes, **caractérisé en ce que** les dispositifs électriques et/ou électroniques (110, 120) sont couplés électriquement et/ou avec transmission de données les uns aux autres.

12. Système selon la revendication 11, **caractérisé en ce que** tous les dispositifs électriques et/ou électroniques (110, 120) sont connectés à un dispositif de commande (60), le dispositif de commande (60) identifie tous les dispositifs électriques et/ou électroniques (110, 120) et provoque leur activation ou désactivation.

13. Procédé de commande d'un système selon l'une des revendications précédentes, **caractérisé en ce que** tous les dispositifs électriques et/ou électroniques (110, 120) présents dans le système et couplés les uns aux autres sont déclenchés par un dispositif de commande (60), dont l'état et l'étendue fonctionnelle sont interrogés, et de l'énergie et/ou des signaux de données sont transmis aux dispositifs électriques et/ou électroniques (110, 120) respectifs.

14. Procédé selon la revendication 13, **caractérisé en ce que** les états de charge de plusieurs moyens de stockage (115, 125) d'énergie électrique sont détectés et, pendant un processus de charge, l'énergie de charge est distribuée sur la base des états de charge détectés.

15. Procédé selon la revendication 13 ou 14, **caractérisé en ce que** plusieurs moyens de stockage (115, 125) d'énergie électrique sont regroupés pour donner un ensemble, et l'alimentation des dispositifs électriques et/ou électroniques (110, 120) à partir dudit ensemble est distribuée de manière centralisée par le dispositif de commande (60).

16. Procédé selon la revendication 15, **caractérisé en ce que** le moyen de stockage (115, 125) d'énergie électrique ayant l'état de charge le plus élevé est tout d'abord attribué à un consommateur.

17. Procédé selon l'une des revendications 13 à 16, **caractérisé en ce que** les moyens de stockage (115, 125) d'énergie électrique effectuent un échange de charge entre eux.

18. Procédé selon l'une des revendications 13 à 17, **caractérisé en ce que** des fonctions des dispositifs électriques et/ou électroniques (110, 120) sont activées ou désactivées sur la base des composants couplés (10, 11, 12).

19. Procédé selon l'une des revendications 13 à 18, **caractérisé en ce que** les dispositifs électriques et/ou électroniques (110, 120) sont authentifiés après le couplage des composants (10, 11, 12), et leur étendue fonctionnelle et/ou leur libération d'énergie est déterminée en fonction des dispositifs électriques et/ou électroniques (110, 120) combinés entre eux.
